# EUROPEAN PATENT APPLICATION

(11) **EP 3 053 594 A1**
(43) Date of publication of application: **10.08.2016**
(21) Application number: 14850464.0
(22) Date of filing: 02.10.2014
(51) Int. Cl.: A61K 39/145, A61K 9/19, A61K 47/26, A61P 31/16

(54) **DRIED INFLUENZA VACCINE PREPARATION AND METHOD FOR PRODUCING DRIED INFLUENZA VACCINE PREPARATION**

(30) Priority: 03.10.2013 JP 2013208661
(71) Applicant: Nitto Denko Corporation, Ibaraki-shi, Osaka 567-8680 (JP)
(72) Inventor: KIYOTOH, Eiji, Ibaraki-shi Osaka 567-8680 (JP); HORI, Mitsuhiko, Ibaraki-shi Osaka 567-8680 (JP); ASARI, Daisuke, Ibaraki-shi Osaka 567-8680 (JP); OKAZAKI, Arimichi, Ibaraki-shi Osaka 567-8680 (JP); FUKASAKA, Masahiro, Ibaraki-shi Osaka 567-8680 (JP)
(74) Representative: Müller-Boré & Partner Patentanwälte PartG mbB
(86) International application number: PCT/JP2014/076344
(87) International publication number: WO 2015/050177

(57) **Abstract**

The present invention provides a dried influenza vaccine preparation in which the activity of an influenza vaccine antigen can be stably maintained even when stored without strictly maintaining a low temperature, and which can be stably supplied. The present invention also provides a method of producing the dried influenza vaccine preparation. The present invention provides a dried influenza vaccine preparation containing an influenza vaccine antigen and a disaccharide, wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

## Description

### TECHNICAL FIELD

The present invention relates to a dried preparation containing an influenza vaccine antigen. More specifically, the present invention relates to a dried influenza vaccine preparation in which the activity of an influenza vaccine antigen can be stably maintained even when stored without strictly maintaining a low temperature, and which can be stably supplied. The present invention also relates to a method of producing the dried influenza vaccine preparation.

### BACKGROUND ART

Influenza is a type of acute infection caused by an influenza virus. The incubation period from infection with the influenza virus to onset of influenza is usually one to two days. The onset is accompanied by the following symptoms, for example: a fever of 38 degrees or higher, systemic symptoms (such as general malaise, headache, joint pain, and muscle pain), sore throat, cough, and nasal discharge. In general, recovery takes one week or less. Influenza may lead to complications such as pneumonia and bronchitis, which may become severe and result in death, in the case of onset of influenza in people such as elderly people, infants, pregnant women, patients with chronic respiratory disease, patients with chronic cardiovascular disease, diabetic patients, and chronic renal failure patients. In addition, influenza intensively occurs in epidemics in a short period of time, and thus sometimes affects the society and causes an economic loss.

Administration of influenza vaccine is the most effective method of preventing influenza from becoming severe. An influenza vaccine preparation is usually a liquid preparation used as an injectable drug or nasal preparation.

For distribution of a liquid preparation of influenza vaccine, a low temperature must be maintained throughout the entire process of distribution and storage (so-called a cold chain) in order to prevent deactivation of an influenza vaccine antigen. Although the epidemic season is different depending on the region, influenza is pandemic, and it is difficult to distribute the preparation while maintaining the activity of the influenza vaccine antigen in the countries and regions where it is difficult to maintain a low temperature.

Currently available influenza vaccine antigens are roughly divided into live attenuated influenza vaccine antigens and inactivated influenza vaccine antigens. Further, inactivated influenza vaccine antigens are classified into the following three groups: (1) whole particle vaccine inactivated with formalin; (2) split vaccine antigen obtained by disrupting virus particles with an organic solvent or surfactant and solubilizing lipid envelopes; and (3) subunit vaccine antigen obtained by purifying hemagglutinin antigen (HA antigen) and neuraminidase antigen (NA antigen). Any of these vaccine antigens is usually prepared by disrupting virus particles with an organic solvent or surfactant and isolating or purifying viral proteins depending on the type.

However, while influenza virus particles have a high sterol content and are usually stable, problems such as a time-dependent decrease in the titer occur during a storage period in the case where the vaccine antigen is obtained by disrupting virus particles, removing lipid matter from the virus particles, and isolating or purifying viral proteins. As described above, because the split vaccine antigen and the subunit vaccine antigen are not necessarily stable, a low temperature must be maintained throughout the entire process of distribution and storage in order to maintain the activity of the influenza vaccine antigen.

As a method of overcoming the above-described drawback of the liquid preparation of influenza vaccine, an attempt has been made to produce a preparation in dry form. For example, conventionally, a saccharide is added in order to store live influenza virus. Patent Literature 1 discloses a method of adding trehalose to live influenza virus, lyophilizing the mixture, and storing the lyophilized mixture. Non-Patent Literature 1 discloses a method of adding lactose or trehalose to form a preparation when lyophilizing an inactivated whole-particle vaccine antigen of influenza virus. Non-Patent Literature 2 discloses a method of adding trehalose to inactivated avian influenza virus, and lyophilizing and storing the virus.

Yet, at present, no dried influenza vaccine preparation having excellent stability is commercially available, and there is a demand for further improvement in stability. In addition, trehalose has been substantially exclusively supplied by Hayashibara Co., Ltd. since the company established a method of producing trehalose at low cost in 1994. Trehalose is used worldwide in various applications, and a monopoly by one company raises concerns over a stable supply. In fact, in 2011, Hayashibara Co. , Ltd. filed for protection under the bankruptcy law, which endangered the supply of trehalose.

### CITATION LIST

### - Patent Literature

Patent Literature 1: JP-T H02-503266

### - Non-Patent Literature

Non-Patent Literature 1: Robert J. Garmise et al. , AAPS Pharm. Sci. Tech., 2006; 7 (1) Article 19, E1-E7
Non-Patent Literature 2: M. S. Pizzuto et al., Biologicals Vol. 39 (2011), 149-151

### SUMMARY OF INVENTION

### - Technical Problem

In view of the above-described situation, the present invention aims to provide a dried influenza vaccine preparation in which the activity of an influenza vaccine antigen can be stably maintained even when stored without strictly maintaining a low temperature, and which can be stably supplied. The present invention also aims to provide a method of producing the dried influenza vaccine preparation.

### - Solution to Problem

In order to solve the above-described problem, the present inventors conducted intensive examinations, and as a result, they found that if the influenza vaccine antigen is prepared in form of a dried preparation in which an influenza vaccine antigen is stabilized by particularly at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol among various additives, the activity of the influenza vaccine antigen can be stably maintained even when the preparation is stored without strictly maintaining a low temperature, unlike the case of conventional liquid preparations. In addition, the dried influenza vaccine preparation as described above is prepared in such a manner that the influenza vaccine antigen is stabilized by a disaccharide that can be stably supplied at low cost. Thus, such a dried influenza vaccine preparation can also be stably supplied.

In other words, the present invention provides a dried influenza vaccine preparation containing an influenza vaccine antigen and a disaccharide, wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

The amount of the disaccharide is preferably 40 to 600 parts by weight per part by weight of HA antigen in the influenza vaccine antigen.

The influenza vaccine antigen is preferably a split vaccine antigen or subunit vaccine antigen, with the split vaccine antigen being more preferred.

The disaccharide is preferably at least one selected from the group consisting of sucrose, maltose, palatinose, and melibiose.

The present invention also provides a method of producing a dried influenza vaccine preparation, the method including drying an influenza vaccine antigen-containing aqueous solution containing an influenza vaccine antigen and a disaccharide under non-thermal conditions, wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

The amount of the disaccharide in the influenza vaccine antigen-containing aqueous solution is preferably 2 to 30% w/v.

The method of drying under non-thermal conditions is preferably a reduced-pressure drying method or lyophilization method.

The present invention is described in detail below.

The dried influenza vaccine preparation of the present invention contains an influenza vaccine antigen.

The "dried preparation" as mentioned herein refers to a preparation having a moisture content of 20 wt% or less.

The strain of influenza virus used in the influenza vaccine antigen is not particularly limited. Examples include an influenza A virus strain and an influenza B virus strain.

The influenza vaccine antigen is not particularly limited. Yet, it is preferably a split vaccine antigen or subunit vaccine antigen prepared in the following manner: growing virus particles in embryonated eggs; disrupting the virus particles with an organic solvent or surfactant; and isolating or purifying viral proteins depending on the type. The split vaccine antigen is more preferred.

The type of the split vaccine antigen is not particularly limited. Examples include hemagglutinin (HA) antigen, neuraminidase (NA) antigen, matrix (M1) antigen, matrix (M2) antigen, and nucleoprotein (NP) antigen. Among these, the hemagglutinin (HA) antigen is preferred in view of effectively inducing immunity by administration of the dried influenza vaccine preparation.

The influenza vaccine antigen may contain a single viral antigen or two or more viral antigens.

A method of producing the influenza vaccine antigen is not particularly limited, and any conventionally known method can be used. For example, the influenza vaccine antigen may be produced from a purified virus stock solution prepared by infecting chicken eggs, cells, or the like by a usual method with virus strains isolated from patients with influenza or animals infected with influenza and culturing the virus strains. Alternatively, the influenza vaccine antigen may be produced from a genetically engineered recombinant virus or specific antigen produced in various cells.

The dried influenza vaccine preparation of the present invention contains a disaccharide.

The disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol. These disaccharides stabilize the influenza vaccine antigen and dissolve well in water. Thus, these disaccharides are easily added to the influenza vaccine antigen-containing aqueous solution that is used to produce the dried influenza vaccine preparation.

The disaccharide is preferably at least one selected from the group consisting of sucrose, maltose, palatinose, and melibiose.

The amount of the disaccharide is preferably less than 700 parts by weight per part by weight of HA antigen in the influenza vaccine antigen, and is more preferably 40 to 600 parts by weight. If the amount is less than 40 parts by weight, the stabilizing effect due to the disaccharide may be insufficient. If the amount is more than 600 parts by weight, the dried influenza vaccine preparation will have high hygroscopic property, and the activity of the influenza vaccine antigen may decrease if the preparation is stored without strictly maintaining a low temperature. A more preferred lower limit of the amount is 80 parts by weight, and a more preferred upper limit thereof is 560 parts by weight. A particularly preferred lower limit is 400 parts by weight, and a particularly preferred upper limit is 500 parts by weight.

The moisture content of the dried influenza vaccine preparation of the present invention is not particularly limited as long as it is 20 wt% or less. Yet, it is preferably 10 wt% or less. If the moisture content is more than 20 wt%, the activity of the influenza vaccine antigen may decrease if the preparation is stored without strictly maintaining a low temperature.

The "moisture content" as mentioned herein is determined in accordance with the Japanese Pharmacopoeia Sixteenth Edition, General Test, Loss on Drying Test, Method 1. In other words, the moisture content is determined from the weight reduction rate of a test piece of the dried influenza vaccine preparation of the present invention when the test piece is heated at 105°C for three hours.

The activity of the influenza vaccine antigen in the dried influenza vaccine preparation of the present invention can be stably maintained even when the preparation is stored without strictly maintaining a low temperature, and thus the preparation can be easily distributed and stored, compared to conventional liquid preparations.

The activity of the influenza vaccine antigen in the dried influenza vaccine preparation of the present invention can be stably maintained even when the preparation is stored, for example, at a storage temperature of 0°C to 50°C. A more preferred limit of the storage temperature is 2°C, and a more preferred upper limit thereof is 40°C.

A preferred method of producing the dried influenza vaccine preparation of the present invention is one in which an influenza vaccine antigen-containing aqueous solution containing the influenza vaccine antigen and the disaccharide is dried under non-thermal conditions. Drying under non-thermal conditions is preferred because the influenza vaccine antigen is thermally unstable.

In other words, the present invention provides a method of producing a dried influenza vaccine preparation, the method including drying an influenza vaccine antigen-containing aqueous solution containing an influenza vaccine antigen and a disaccharide under non-thermal conditions, wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

The amount of the influenza vaccine antigen in the influenza vaccine antigen-containing aqueous solution is preferably 100 µg HA/mL or more. If the amount is less than 100 µg HA/mL, the dried influenza vaccine preparation may have poor stability. A more preferred lower limit of the amount is 200 µg HA/mL.

An upper limit of the amount of the influenza vaccine antigen in the influenza vaccine antigen-containing aqueous solution is not particularly limited, and the amount may exceed 1000 µg HA/mL.

The amount of the disaccharide in the influenza vaccine antigen-containing aqueous solution is preferably less than 35% w/v, more preferably 2 to 30% w/v. If the amount is less than 2% w/v, the stabilizing effect due to the disaccharide may be insufficient. If the amount is more than 30% w/v, the dried influenza vaccine preparation will have high hygroscopic property, and the activity of the influenza vaccine antigen may decrease if the preparation is stored without strictly maintaining a low temperature. In addition, crystallization of the disaccharide may be promoted, decreasing the activity of the influenza vaccine antigen. A more preferred lower limit of the amount is 4% w/v, and a more preferred upper limit is 28% w/v. A particularly preferred lower limit is 20% w/v, and a particularly preferred upper limit is 25% w/v.

The method of drying under non-thermal conditions is not particularly limited. Yet, it is preferably a reduced-pressure drying method or lyophilization method, with the lyophilization method being particularly preferred. The lyophilization method is not particularly limited. Any method that uses a conventionally known lyophilization device can be used.

### - Advantageous Effects of Invention

The activity of the influenza vaccine antigen in the dried influenza vaccine preparation of the present invention can be stably maintained even when the preparation is stored without strictly maintaining a low temperature, and thus the preparation can be easily distributed and stored, compared to conventional liquid preparations.

In addition, the dried influenza vaccine preparation of the present invention is prepared in such a manner that the influenza vaccine antigen is stabilized by a disaccharide that can be stably supplied at low cost. Thus, the dried influenza vaccine preparation of the present invention can also be stably supplied.

Further, the dried influenza vaccine preparation of the present invention can be used as it is or by being dissolved or dispersed in a solvent that can be administered to a living body (such as a normal saline solution or water for injection) when used. Thus, the preparation can be used in various administration forms. Specifically, the preparation can be used as an injectable drug or a mucosal administration preparation to be administered to nasal mucous membrane, intraoral mucous membrane, ocular mucous membrane, ear mucous membrane, genital mucous membrane, pharynx mucous membrane, respiratory tract mucous membrane, bronchial mucous membrane, pulmonary mucous membrane, gastric mucous membrane, intestinal mucous membrane, or rectal mucous membrane.

### DESCRIPTION OF EMBODIMENTS

The present invention is described in more detail below with reference to examples, but is not limited to these examples.

### (Example 1)

### (Lyophilized influenza HA vaccine preparation)

Sucrose (produced by Wako Pure Chemical Industries, Ltd.) shown in Table 1 was added to influenza HA antigen (type A H1N1 : A/California/07/2009 produced by the Research Foundation for Microbial Diseases of Osaka University), and then PBS for adjustment (phosphate-buffered sodium chloride solution) having the composition described below was added to the mixture to prepare an influenza vaccine antigen-containing aqueous solution containing 20% w/v of sucrose and 500 µg HA/mL of influenza HA antigen (400 parts by weight of sucrose per part by weight of influenza HA antigen). The thus-obtained influenza vaccine antigen-containing aqueous solution (10 µL) was dispensed in a 1. 5-mL safe-lock tube (produced by Eppendorf) and lyophilized.

### PBS for adjustment

Sodium chloride (produced by Wako Pure Chemical Industries, Ltd.) 4.25 g
Disodium hydrogen phosphate 12-hydrate (produced by Wako Pure Chemical Industries, Ltd.) 1.76 g
Sodium dihydrogen phosphate dihydrate (produced by Wako Pure Chemical Industries, Ltd.) 0.35 g
Distilled water filled up to 500 mL in a measuring flask

### (Comparative Example 1)

### (Liquid influenza HA vaccine preparation)

A liquid influenza HA vaccine preparation was obtained in the same manner as in Example 1 except that the influenza vaccine antigen-containing aqueous solution was used as it is without addition of the disaccharide and without lyophilization.

### (Comparative Example 2)

### (Lyophilized influenza HA vaccine preparation)

A lyophilized influenza HA vaccine preparation was obtained in the same manner as in Example 1 except that the disaccharide was not added.

### <Measurement Example 1: Measurement of the activity of the influenza HA vaccine>

### (1) Preparation of a chicken erythrocyte suspension (1 vol%)

Preserved chicken blood (produced by Nippon Biotest Laboratories Inc.) was placed in a centrifuge tube and centrifuged at 900 G for five minutes. Subsequently, the supernatant and the leukocyte layer were removed. Next, PBS for dilution (a phosphate-buffered sodium chloride solution (pH 7.2)) having the composition described below was added to the erythrocytes in the centrifuge tube, followed by stirring. Then, centrifugation was performed to remove the supernatant. This operation was repeated three times. The erythrocytes in the centrifuge tube were separated out and mixed into a vessel containing a dilute solution. Thereby, a chicken erythrocytes suspension (1 vol%) was prepared.

### PBS for dilution

Sodium chloride (produced by Wako Pure Chemical Industries, Ltd.) 8.5 g
Disodium hydrogen phosphate 12-hydrate (produced by Wako Pure Chemical Industries, Ltd.) 1.425 g
Sodium dihydrogen phosphate dihydrate (produced by Wako Pure Chemical Industries, Ltd.) 0.135 g
Distilled water filled up to 1000 mL in a measuring flask

### (2) Measurement of the activity

A total of 50 µL of an influenza HA vaccine preparation (1. 2-fold dilution series) was placed in the wells of a V-bottom microplate. Subsequently, 50 µL of the chicken erythrocyte suspension (1 vol%) was added thereto. The mixture was mixed well by pipetting and allowed to stand at room temperature for one hour. The final dilution rate of the influenza HA vaccine preparation in which the erythrocytes were completely agglutinated was regarded as the HA titer.

Table 1 shows relative values (%), assuming that the HA titer of the liquid influenza HA vaccine preparation of Comparative Example 1 obtained without addition of the disaccharide and without lyophilization and stored at 5°C as 100%.

**[Table 1]**

| | Influenza vaccine antigen-containing aqueous solution | | HA antigen : disaccharide (weight ratio) | Amount of liquid before lyophilization (*µ*L) | Lyophilization | Measurement of the activity |
|---|---|---|---|---|---|---|
| | Influenza HA antigen (*µ*g HA/mL) | Disaccharide (% w/v) | | | | Immediately after preparation |
| | Type A H1N1 | Sucrose | | | | |
| Example 1 | 500 | 20 | 1:400 | 10 | Done | 100% |
| Comparative Example 1 | 500 | - | - | 10 | Not applicable (liquid form) | 100% |
| Comparative Example 2 | | | | | Done | 13% |

The results in Table 1 confirmed that the activity markedly decreases in the case where the influenza HA antigen is lyophilized without adding anything, whereas the HA titer is stably maintained when the influenza HA antigen is mixed with 20% w/v of sucrose and lyophilized.

### (Examples 2 to 17)

### (Lyophilized influenza HA vaccine preparation)

A lyophilized influenza HA vaccine preparation was obtained in the same manner as in Example 1 except that the type or amount of disaccharide or the amount of liquid before lyophilization was changed as shown in Table 2.

### (Comparative Example 3)

### (Liquid influenza HA vaccine preparation)

A liquid influenza HA vaccine preparation was obtained in the same manner as in Example 1 except that the influenza vaccine antigen-containing aqueous solution was used as it is without lyophilization.

### (Comparative Examples 4 to 7)

### (Lyophilized influenza HA vaccine preparation)

A lyophilized influenza HA vaccine preparation was obtained in the same manner as in Example 1 except that a monosaccharide or a trisaccharide shown in Table 4 was used instead of the disaccharide.

### (Examples 18 to 21)

### (Lyophilized influenza HA vaccine preparation)

A lyophilized influenza HA vaccine preparation was obtained in the same manner as in Example 1 except that the amount of the influenza HA antigen was changed as shown in Table 5.

### (Examples 22, 23, and 24)

### (Lyophilized influenza HA vaccine preparation)

A lyophilized influenza HA vaccine preparation was obtained in the same manner as in Example 1 except that the type and amount of the influenza HA antigen were changed as shown in Table 6.

### (Comparative Examples 8, 9, and 10)

### (Liquid influenza HA vaccine preparation)

A liquid influenza HA vaccine preparation was obtained in the same manner as in Examples 22, 23 and 24 except that the influenza vaccine antigen-containing aqueous solution was used as it is without addition of the disaccharide and without lyophilization.

### <Measurement example 2: Measurement of the activity of the influenza HA vaccine after storage>

The lid of a tube containing the influenza HA vaccine preparation was closed. Then, the tube was placed in an airtight container with a desiccant and stored at 30°C or 40°C for one month.

In Examples 1 to 9 and 12 to 17 and Comparative Examples 1 to 7, 1000 µL of the PBS for adjustment having the above composition was added to redissolve the influenza HA vaccine preparation, and the HA titer of the influenza HA vaccine preparation was measured in the same manner as in Measurement Example 1.

In Examples 10 and 11, 50 µL of distilled water was added to redissolve the influenza HA vaccine preparation, and the PBS for adjustment having the above composition was added to make a total of 1000 µL. Then, the HA titer of the influenza HA vaccine preparation was measured in the same manner as in Measurement Example 1.

In Examples 18, 19, 20, and 21, the PBS for adjustment having the above composition was added, respectively, in amounts of 800 µL, 600 µL, 400 µL, and 200 µL to redissolve the influenza HA vaccine preparation, and the HA titer of the influenza HA vaccine preparation was measured in the same manner as in Measurement Example 1.

In Example 22 and Comparative Example 8; Example 23 and Comparative Example 9; and Example 24 and Comparative Example 10, the PBS for adjustment having the above composition was added, respectively, in amounts of 300 µL (type A H3N2), 1200 µL (type B Victoria lineage), and 20000 µL (type B Yamagata lineage) to redissolve the influenza HA vaccine preparation. Then, the HA titer of the influenza HA vaccine preparation was measured in the same manner as in Measurement Example 1.

Tables 2 to 6 show relative values (%), assuming that the HA titer of the liquid influenza HA vaccine preparation of Comparative Example 1 obtained without addition of the disaccharide and without lyophilization and stored at 5°C as 100%.

**[Table 2]**

| | Influenza vaccine antigen-containing aqueous solution | | | | | HA antigen : disaccharide (weight ratio) | Amount of liquid before lyophilization. (*µ* L) | Lyophilization | Storage temp. | Measurement of the activity | | |
|---|---|---|---|---|---|---|---|---|---|---|---|---|
| | Influenza HA antigen (*µ* g HA/mL) | Disaccharide (% w/v) | | | | | | | | Immediately after preparation | After two weeks | After one month |
| | Type AH1N1 | Sucrose | Maltose | Melibiose | Palatinose | | | | | | | |
| Example 1 | | 20 | - | - | - | 1:400 | | | 30°C | 100% | 100% | 100% |
| | | | | | | | | | 40°C | 100% | 100% | 100% |
| Example 2 | | 2 | - | - | - | 1:40 | | | 30°C | 83% | 83% | 74% |
| | | | | | | | | | 40°C | 83% | 83% | 66% |
| Example 3 | | 5 | - | - | - | 1:100 | | | 30°C | 83% | 100% | 83% |
| | | | | | | | | | 40°C | 83% | 100% | 83% |
| Example 4 | | 10 | - | - | - | 1:200 | | | 30°C | 83% | 100% | 89% |
| | | | | | | | | | 40°C | 83% | 100% | 94% |
| Example 5 | 500 | 15 | - | - | - | 1:300 | 10 | Done | 30°C | 100% | 100% | 89% |
| | | | | | | | | | 40°C | 100% | 100% | 89% |
| Example 6 | | 25 | - | - | - | 1:500 | | | 30°C | 100% | 100% | 100% |
| | | | | | | | | | 40°C | 100% | 100% | 100% |
| Example 7 | | 30 | - | - | - | 1:600 | | | 30°C | 100% | 78% | 94% |
| | | | | | | | | | 40°C | 100% | 55% | 38% |
| Example 8 | | 35 | - | - | - | 1:700 | | | 30°C | 100% | 100% | 67% |
| | | | | | | | | | 40°C | 100% | 19% | 7% |
| Example 9 | | 50 | - | - | - | 1:1000 | | | 30°C | 94% | 48% | 89% |
| | | | | | | | | | 40°C | 94% | 42% | 10% |
| Example 10 | 500 | 20 | - | - | - | 1:400 | 50 | Done | 30°C | 100% | 100% | 100% |
| | | | | | | | | | 40°C | 100% | 100% | 100% |
| Example 11 | | 4 | - | - | - | 1:80 | | | 30°C | 100% | 100% | 83% |
| | | | | | | | | | 40°C | 100% | 100% | 83% |
| Example 12 | 500 | - | 10 | - | - | 1:200 | 10 | Done | 30°C | 100% | 100% | 100% |
| | | | | | | | | | 40°C | 100% | 100% | 100% |
| Example 13 | | - | 20 | - | - | 1:400 | | | 30°C | 100% | 100% | 100% |
| | | | | | | | | | 40°C | 100% | 94% | 84% |
| Example 14 | | - | 30 | - | - | 1:600 | | | 30°C | 100% | 100% | 100% |
| | | | | | | | | | 40°C | 100% | 100% | 100% |
| Example 15 | | - | 40 | - | - | 1:800 | | | 30°C | 100% | 100% | 100% |
| | | | | | | | | | 40°C | 100% | 100% | 100% |
| Example 16 | 500 | - | - | 20 | - | 1:400 | 10 | Done | 30°C | 100% | 100% | 100% |
| | | | | | | | | | 40°C | 100% | 100% | 100% |
| Example 17 | | - | - | - | 20 | 1:400 | | | 30°C | 100% | 100% | 100% |
| | | | | | | | | | 40°C | 100% | 100% | 100% |

The results in Table 2 confirmed that in the case where the influenza HA antigen is mixed with sucrose (produced by Wako Pure Chemical Industries, Ltd.), maltose (produced by Wako Pure Chemical Industries, Ltd.), melibiose (produced by Wako Pure Chemical Industries, Ltd.), or palatinose (produced by Wako Pure Chemical Industries, Ltd.) and lyophilized, the HA titer is stably maintained even when the preparation is stored at 40°C for one month.

When adding sucrose, it was confirmed that the HA titer is most stably maintained in the case where the influenza HA antigen is mixed with 20 to 25% w/v of sucrose (400 to 500 parts by weight of sucrose per part by weight of influenza HA antigen) and lyophilized (Examples 1 and 6). When adding maltose, it was confirmed that the HA titer is most stably maintained in the case where the influenza HA antigen is mixed with 10 to 40% w/v of maltose (200 to 800 parts by weight of maltose per part by weight of influenza HA antigen) and lyophilized.

It was also confirmed that the HA titer is stably maintained even when the amount of liquid before lyophilization is changed (Examples 10 and 11).

**[Table 3]**

| | Influenza vaccine antigen-containing aqueous solution | | HA antigen : disaccharide (weight ratio) | Amount of liquid before lyophilization (*µ* L) | Lyophilization | Storage temp. | Measurement of the activity | | |
|---|---|---|---|---|---|---|---|---|---|
| | Influenza HA antigen (*µ* g HA/mL) | Disaccharide (% w/v) | | | | | Immediately after preparation | After two weeks | After one month |
| | Type A H1N1 | Sucrose | | | | | | | |
| Comparative Example 1 | 500 | - | - | 10 | Not applicable (liquid form) | 5°C | 100% | 100% | 100% |
| | | | | | | 30°C | 100% | 83% | 79% |
| | | | | | | 40°C | 100% | 51% | 5% |
| Comparative Example 2 | | | | | Done | 30°C | 9% | 5% | 5% |
| | | | | | | 40°C | 9% | 5% | 5% |
| Comparative Example 3 | 500 | 20 | 1:400 | 10 | Not applicable (liquid form) | 30°C | 100% | 83% | 79% |
| | | | | | | 40°C | 100% | 69% | 33% |

The results in Table 3 confirmed that the activity markedly decreases in the case where the influenza HA antigen is lyophilized without adding anything (Comparative Example 2). It was also confirmed that the HA titer decreases over time in the case where the influenza HA antigen containing 20% w/v of sucrose is stored as it is in liquid form at 40°C for one month (Comparative Example 3).

**[Table 4]**

| | Influenza vaccine antigen-containing aqueous solution | | | | | HA antigen : disaccharide (weight ratio) | Amount of liquid before lyophilization (*µ* L) | Lyophilization | Storage temp. | Measurement of the activity | | |
|---|---|---|---|---|---|---|---|---|---|---|---|---|
| | Influenza HA antigen (*µ*g HA/mL) | Monosaccharide (% w/v) | | | Trisaccharide (% w/v) | | | | | Immediately after preparation | After two weeks | After one month |
| | Type AH1N1 | Glucose | Galactose | Fructose | Faffinose | | | | | | | |
| Comparative Example 4 | | 20 | - | - | - | 1:400 | | | 30°C | 100% | 66% | 38% |
| | | | | | | | | | 40°C | 100% | 36% | 29% |
| Comparative Example 5 | | - | 20 | - | - | 1:400 | | | 30°C | 100% | 11% | 13% |
| | 500 | | | | | | 10 | Done | 40°C | 100% | 5% | 8% |
| Comparative Example 6 | | - | - | 20 | - | 1:400 | | | 30°C | 100% | 83% | 94% |
| | | | | | | | | | 40°C | 100% | 27% | 32% |
| Comparative Example 7 | | - | - | - | 20 | 1:400 | | | 30°C | 89% | 74% | 58% |
| | | | | | | | | | 40°C | 89% | 79% | 55% |

The results in Table 4 confirmed that the HA titer decreases in the case where the influenza HA antigen is mixed with 20% w/v of monosaccharide (glucose (produced by Wako Pure Chemical Industries, Ltd.), galactose (produced by Wako Pure Chemical Industries, Ltd.) or fructose (produced by Wako Pure Chemical Industries, Ltd.)) or a trisaccharide (raffinose (produced by Wako Pure Chemical Industries, Ltd.)) and lyophilized.

**[Table 5]**

| | Influenza vaccine antigen-containing aqueous solution | | HA antigen : disaccharide (weight ratio) | Amount of liquid before lyophilization (*µ* L) | Lyophilization | Storage temp. | Measurement of the activity | | |
|---|---|---|---|---|---|---|---|---|---|
| | Influenza HA antigen (*µ* g HA/mL) | Disaccharide (% w/v) | | | | | Immediately after preparation | After two weeks | After one month |
| | Type A H1N1 | Sucrose | | | | | | | |
| Example 18 | 400 | | 1:500 | | | 30°C | 100% | 100% | 100% |
| | | | | | | 40°C | 100% | 100% | 100% |
| Example 19 | 300 | | 1:667 | | | 30°C | 100% | 100% | 100% |
| | | 20 | | 10 | Done | 40°C | 100% | 100% | 100% |
| Example 20 | 200 | | 1:1000 | | | 30°C | 100% | 100% | 100% |
| | | | | | | 40°C | 100% | 100% | 100% |
| Example 21 | 100 | | 1:2000 | | | 30°C | 83% | 89% | 84% |
| | | | | | | 40°C | 83% | 83% | 83% |

The results in Table 5 confirmed that in the case where the influenza HA antigen is mixed with 20% w/v of sucrose and lyophilized, the HA titer is stably maintained at any amount of the influenza HA antigen in the range of 100 to 400 µg HA/mL (500 to 2000 parts by weight of sucrose per part by weight of influenza HA antigen), even when the preparation is stored at 40°C for one month.

**[Table 6]**

| | Influenza vaccine antigen-containing aqueous solution | | | | HA antigen: disaccharide (weight ratio) | Amount of liquid before lyophilization (*µ* L) | Lyophilization | Storage temp. | Measurement of the activity | | |
|---|---|---|---|---|---|---|---|---|---|---|---|
| | Influenza HA antigen (*µ* g HA/mL) | | | Disaccharide (% w/v) | | | | | Immediately after preparation | After two weeks | After one month |
| | Type A H3N2 | Type B | | Sucrose | | | | | | | |
| | | Victoria lineage | Yamagata lineage | | | | | | | | |
| Comparative Example 8 | 400 | - | - | - | - | 10 | Not applicable (liquid form) | 5°C | 100% | 100% | 100% |
| Example 22 | 400 | - | - | 20 | 1:500 | 10 | Done | 30°C | 100% | 100% | 83% |
| | | | | | | | | 40°C | 100% | 94% | 83% |
| Comparative Example 9 | - | 300 | - | - | - | 10 | Not applicable (liquid form) | 5°C | 100% | 100% | 100% |
| Example 23 | - | 300 | - | 20 | 1:667 | 10 | Done | 30°C | 100% | 100% | 100% |
| | | | | | | | | 40°C | 100% | 100% | 100% |
| Comparative Example 10 | - | - | 400 | - | - | 10 | Not applicable (liquid form) | 5°C | 100% | 100% | 100% |
| Example 24 | - | - | 400 | 20 | 1:500 | 10 | Done | 30°C | 100% | 100% | 100% |
| | | | | | | | | 40°C | 100% | 100% | 100% |

The results in Table 6 confirmed that, as is the case with type A H1N1, in any of type A H3N2 (A/Victoria/361/2011 produced by the Research Foundation for Microbial Diseases of Osaka University) and type B (B/Brisbane/60/2008 (Victoria lineage) or B/Wisconsin/1/2010 (Yamagata lineage) produced by the Research Foundation for Microbial Diseases of Osaka University), the HA titer is stably maintained even when the preparation is stored at 40°C for one month, in the case where the influenza HA antigen is mixed with 20% w/v of sucrose (500 parts by weight of sucrose per part by weight of type A H3N2; 667 parts by weight of sucrose per part by weight of type B (Victoria lineage); or 500 parts by weight of sucrose per part by weight of type B (Yamagata lineage)) and lyophilized.

### INDUSTRIAL APPLICABILITY

The present invention provides a dried influenza vaccine preparation in which the activity of an influenza vaccine antigen can be stably maintained even when stored without strictly maintaining a low temperature, and which can be stably supplied. The present invention also provides a method of producing the dried influenza vaccine preparation.

## Claims

1. A dried influenza vaccine preparation comprising an influenza vaccine antigen and a disaccharide,
wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

2. The dried influenza vaccine preparation according to claim 1,
wherein an amount of the disaccharide is 40 to 600 parts by weight per part by weight of HA antigen in the influenza vaccine antigen.

3. The dried influenza vaccine preparation according to claim 1 or 2,
wherein the influenza vaccine antigen is a split vaccine antigen or subunit vaccine antigen.

4. The dried influenza vaccine preparation according to claim 3,
wherein the influenza vaccine antigen is a split vaccine antigen.

5. The dried influenza vaccine preparation according to claim 1, 2, 3, or 4,
wherein the disaccharide is at least one selected from the group consisting of sucrose, maltose, palatinose, and melibiose.

6. A method of producing a dried influenza vaccine preparation, the method comprising drying an influenza vaccine antigen-containing aqueous solution containing an influenza vaccine antigen and a disaccharide under non-thermal conditions,
wherein the disaccharide is at least one selected from the group consisting of sucrose, maltose, palatinose, melibiose, isomalt, cellobiose, allolactose, isomaltose, sophorose, lactobionic acid, laminaribiose, xylobiose, turanose, gentiobiose, rutinose, kojibiose, nigerose, robinose, neohesperidose, sucralose, and maltitol.

7. The method of producing a dried influenza vaccine preparation according to claim 6,
wherein an amount of the disaccharide in the influenza vaccine antigen-containing aqueous solution is 2 to 30% w/v.

8. The method of producing a dried influenza vaccine preparation according to claim 6 or 7,
wherein the method of drying under non-thermal conditions is a reduced-pressure drying method or lyophilization method.
